Europäisches Patentamt

European Patent Office

Office européen des brevets

(11) Veröffentlichungsnummer: **0 096 422**
**A2**

## (12) EUROPÄISCHE PATENTANMELDUNG

(21) Anmeldenummer: **83105668.4**

(22) Anmeldetag: **09.06.83**

(51) Int. Cl.³: **A 61 K 39/015**

(30) Priorität: **09.06.82 DE 3221881**

(43) Veröffentlichungstag der Anmeldung:
**21.12.83 Patentblatt 83/51**

(84) Benannte Vertragsstaaten:
**IT**

(71) Anmelder: **Münchener Apparatebau für elektronische Geräte KIMMEL GmbH**
**Hans-Stiessberger Strasse 2 Postfach 1220**
**D-8013 Haar(DE)**

(72) Erfinder: **Jung, Albrecht, Dr.**
**Hausserstrasse 142**
**D-7400 Tübingen(DE)**

(74) Vertreter: **Weickmann, Heinrich, Dipl.-Ing. et al,**
**Patentanwälte Dipl.-Ing. H.Weickmann Dipl.-Phys.Dr. K. Fincke Dipl.-Ing. F.A. Weickmann Dipl.-Chem. B. Huber Dr.-Ing. H. Liska Dipl.-Phys.Dr. J. Prechtel Möhlstrasse 22**
**D-8000 München 86(DE)**

(54) **Verfahren zur Herstellung eines Impfstoffs gegen parasitäre Krankheitserreger.**

(57) Zur Herstellung eines Impfstoffes gegen Parasiten, insbesondere Malariaparasiten, läßt man mit dem Parasiten infizierte Erythrocyten in vivo oder in vitro unter Hypoferrämiebedingungen, beispielsweise Zusatz eines Eisenchelatbildners, wachsen, isoliert die Parasiten aus den Erythrocyten und verwendet die isolierten Parasiten oder Zellwandfragmente derselben als Antigen zur Gewinnung von Antiserum.

EP 0 096 422 A2

B e s c h r e i b u n g

Die Erfindung betrifft ein Verfahren zur Herstellung
eines Impfstoffes gegen Parasiten, insbesondere gegen
Malariaparasiten.

Es ist bekannt, Impfstoffe durch Infektion eines Organismus mit Parasiten als Antigen und Gewinnung von
Antiserum aus dem infizierten Organismus zu gewinnen.
Ebenso ist es bekannt, veränderte Parasiten, welche wesentlich herabgesetzte Virulenz aufweisen, als Aktivimpfstoff direkt einzusetzen. In beiden Fällen wird im
infizierten Organismus die Bildung von Antikörpern induziert, die im einen Falle im Form von Antiserum zur
Passivimpfung verwendet werden, im anderen Falle den
Organismus gegen die Infektion durch virulente Parasiten schützen.

Bei vielen Parasiten, insbesondere aber bei Malariaparasiten und phylogenetisch damit verwandten Parasiten
erwies sich dieses bekannte Verfahren als unwirksam
bzw. ungenügend wirksam. In diesen Fällen ist daher die
chemotherapeutische Behandlung trotz der damit verbundenen bekannten Nachteile immer noch das Mittel der
Wahl bei der Prophylaxe oder Behandlung der Parasiteninfektion.

Der Erfindung liegt die Aufgabe zugrunde, diesen Nachteil zu beseitigen und ein Verfahren zu schaffen, welches die Gewinnung eines Impfstoffes sowohl für die
Aktivimpfung als auch für die Passivimpfung gegen Parasiteninfektionen ermöglicht, bei denen bisher Impfstoffe nicht erhalten werden konnten. Eine weitere Aufgabe der Erfindung liegt in der Herstellung von wirksameren Impfstoffen gegen Parasiten, bei denen es schon bisher möglich war, Impfstoffe zu erhalten.

Gelöst wird diese Aufgabe erfindungsgemäß durch ein Verfahren zur Herstellung eines Impfstoffes gegen Parasiten, insbesondere Malariaparasiten, durch Infektion eines Organismus mit den Parasiten als Antigen und Gewinnung von Antiserum aus dem infizierten Organismus, welches dadurch gekennzeichnet ist, daß man mit dem Parasiten infizierte Erythrocyten in vivo oder in vitro unter Hypoferrämiebedingungen wachsen läßt, die Parasiten aus den Erythrocyten isoliert und die isolierten Parasiten oder Zellwandfragmente derselben als Antigen verwendet. Die erfindungsgemäß als Antigen verwendeten Parasiten oder deren Zellwandfragmente weisen eine wesentlich gesteigerte Antigenität auf.

Die Erfindung geht von der Annahme aus, daß das Eisentransportsystem der Parasiten, insbesondere der Malariaparasiten aber auch der phylogenetisch mit diesen verwandten Parasiten, vom Eisentransportsystem der Warmblütlerzelle verschieden ist und daher Bedingungen erzeugt werden können, die zur Bildung von Antikörpern führen, welche anscheinend die für den Eisentransport erforderlichen Regionen in der Parasitenmembran vollständig blockieren, ohne das Eisentransportsystem der Zellen des Wirtsorganismus wesentlich zu stören. Es wird angenommen, daß hierdurch die Zellwand des Parasiten so verändert wird, daß sie die beobachtete gesteigerte Antigenität aufweist.

Unter Hypoferrämiebedingungen (Eisenmangelbedingungen) werden im Rahmen der vorliegenden Erfindung Bedingungen verstanden, welche den Erythrocyten bzw. ihrem Wirtsorganismus weniger Eisenionen zur Verfügung stellen, als dem Mindestbedarf für eine normale Entwicklung entspricht.
Werden die Erythrocyten in vivo gehalten, wird mit
anderen Worten ein Wirtstier mit den Parasiten infiziert, so lassen sich Hypoferrämiebedingungen erzielen,
indem man das Wirtstier mit einer Eisenmangeldiät oder
einer eisenfreien Diät versorgt. Alternativ ist es möglich, der Diät einen Eisenchelatbildner zuzusetzen, der
das in der Diät und im Wirtsorganismus vorhandene Eisen
teilweise oder vollständig bindet. Um die Belastung des
Wirtsorganismus hierbei möglichst gering zu halten,
werden biologische Eisenchelatbildner bevorzugt, da
diese im allgemeinen von der Zelle leicht abgebaut
werden können, insbesondere wenn es sich um Proteine
handelt. Besonders bevorzugt wird als biologischer
Eisenchelatbildner Desferrioxamin, im Handel erhältlich
unter der Bezeichnung Desferal. Beispiele für andere
biologische Eisenchelatbildner, die im Rahmen der
Erfindung verwendet werden können, sind Endotoxin,
Ferritin und Lactoferrin sowie Gemische derselben.
Ebenfalls verwendbar, aber nicht bevorzugt, sind
chemische Eisenchelatbildner, wie Ethylendiamintetraessigsäure und die im chemischen Aufbau ähnlichen
Substanzen. Diese werden jedoch vom Organismus schwieriger abgebaut und sind weniger spezifisch für Eisen.

Auch Resochin erwies sich als Eisenchelatbildner und
kann in unter der kurativen Dosis liegenden Menge eingesetzt werden.

Falls die Züchtung der infizierten Erythrocyten in
vitro erfolgt, setzt man der Nährlösung entsprechend
geringe Eisenmengen oder ein oder mehrere Eisenchelatbildner zu.

Gleichgültig ob die infizierten Erythrocyten in vivo
oder in vitro wachsen gelassen werden, in jedem Fall
wendet man bevorzugt eine Kombination von Eisenmangeldiät und Eisenchelatbildnerzusatz an.

Die Isolierung der Parasiten aus den Erythrocyten kann
nach bekannten Methoden erfolgen. Im Falle von Malariaparasiten eignet sich insbesondere die in Z. Parasitenkd.
58, 151-159 (1979) beschriebene Methode. Sie beruht auf
einer Aggregation der Erythrocyten, z. B. mit Concanavalin A, vorsichtige mechanische Lyse der aggregierten
Erythrocyten, z. B. durch Passieren eines entsprechend
engmaschigen Netzes, und Auftrennung und Separierung
der Parasiten durch trägerfreie Elektrophorese in einer
geeigneten Pufferlösung. Anstelle von Concanavalin A
können auch andere Aggregationsmittel verwendet werden,
wie z. B. Antiseren, Phyt-Häm-agglutümin (PHA) und dergleichen.

Gemäß einer anderen bekannten Methode werden die
Erythrocyten nach quasi Vernetzung mit Hilfe von Lectinen und Lyse durch schwache Scherkräfte in einem
Percoll-Gradienten durch Zentrifugieren bei niedrigen
g-Werten isoliert (10. Internationaler Kongress für
tropische Medizin und Malaria, Manila, 9. bis 15.11.1980).

Die so erhaltenen isolierten Parasiten unterscheiden
sich von den unter Normalbedingungen gewachsenen
Parasiten durch eine wesentlich verstärkte antigene
Wirksamkeit. Diese verstärkte antigene Wirksamkeit
kommt nicht nur dem vollständigen Parasiten sondern
auch dessen durch mechanische oder chemische Lyse gebildeten Zellwandfragmenten zu. Falls ein Antiserum als
Passivimpfstoff hergestellt werden soll, verwendet man
bevorzugt den isolierten vollständigen Parasiten als Antigen. Im Falle einer Aktivimpfung werden bevorzugt
Zellwandfragmente verabreicht. Es können jedoch auch
für die Antiserumgewinnung Zellwandfragmente verwendet
werden und andererseits auch die vollständigen isolierten Parasiten als Aktivimpfstoff eingesetzt werden.

Die Impfung erfolgt in beiden Fällen unter den dem
Fachmann geläufigen Bedingungen. Die Mitverwendung von
Adjuvanz ist nicht erforderlich. Gemäß einer bevorzugten Ausführung der Erfindung wird der hypoferrämische
Parasit bzw. dessen Zellwandfragment jedoch zusammen
mit hypoferrämischem Serum verabreicht. Unter hypoferrämischem Serum wird das Serum von Versuchstieren verstanden, welche unter hypoferrämischen Bedingungen, wie
oben näher erläutert, gehalten wurden. Hierbei kann es
sich sowohl um das Serum von infizierten Wirtstieren
handeln, die für die Bildung von hypoferrämischen Parasiten verwendet wurden, als auch um nichtinfizierte
aber unter Hypoferrämiebedingungen gehaltene Tiere.
Hierdurch läßt sich eine weitere Steigerung der antigenen Wirksamkeit erzielen, die insbesondere bei der Verwendung von Zellwandfragmenten ausgeprägt ist.

Bei dieser bevorzugten Ausführungsform der Erfindung
unter Verwendung eines hypoferrämischen Serums wird
zweckmäßig dem Serum nach der Entnahme aus dem Wirtstier ein Proteinaseinhibitor, wie z. B. Trasylol, zugesetzt.

Erfolgt die Züchtung des hypoferrämischen Parasiten in
Erythrocyten, die in vivo gehalten werden, so hält man
vorzugsweise das Wirtstier vor der Infektion eine gewisse Zeit unter Hypoferrämiebedingungen. Hierbei wird
der Eisenspeicher im Wirtstierorganismus geleert und verhindert, daß nach der Infektion der Parasit noch für
einige Zeit mit ausreichend Eisen versorgt wird oder,
um dies zu verhüten, so große Mengen an Eisenchelatbildner zugesetzt werden, daß auch auf den Wirtsorganismus
selbst der Eisenmangel nachteilig wirkt.

Die Züchtung der hypoferrämischen Parasiten erfolgt
vorzugsweise über eine Mehrzahl von Passagen. Dabei hat
es sich als zweckmäßig erwiesen, nach jeweils etwa 2
bis 8 Passagen durch das Wirtstier bzw. die Erythrocytenkultur den Zusatz an Eisenchelatbildner zur Diät- bzw.
der Nährflüssigkeit zu erhöhen. Gute Ergebnisse werden
in der Regel schon mit 4 bis 10 Passagen erzielt,
jedoch können auch mehr Passagen angewendet werden.

Im Falle der Verwendung von Malariaparasiten reicht im
allgemeinen ein Wachstum von 2 bis 6, vorzugsweise 3
bis 4 Tagen je Passage aus. Gemäß einer besonders bevorzugten Ausführungsform der Erfindung werden daher
Versuchstiere 2 Tage z. B. mit Desferrioxamin vorbehandelt und dann mit den Parasiten infiziert. Nach 3 oder
4 Tagen wird infiziertes Blut entnommen und dann in
gleicher Weise für die jeweils nächste Passage wieder
verwendet.

Die zur Erzeugung der für Hypoferrämiebedingungen
erforderliche Menge an Chelatbildner läßt sich jeweils
experimentell bestimmen. Im Falle des Desferrioxamins
erwies sich ein Bereich von 20 bis 100 mg pro kg pro
Tag bei subcutaner Verabreichung als gut geeignet. Bei
einer weiteren Dosiserhöhung konnte eine Steigerung der
Wirksamkeit nicht festgestellt werden.

- 7 -

Das Verfahren der Erfindung ist, wie erwähnt, auch mit Eisenmangeldiät alleine durchführbar. In diesem Falle wird zweckmäßig während des letzten Teils der Züchtung die Eisenzugabe völlig eingestellt. Hierdurch wird jedoch die erforderliche Wachstumsdauer für die Erzielung der speziellen antigenen Eigenschaften verlängert im Vergleich zu einem wachsen lassen unter Zusatz eines Eisenchelatbildners.

Gemäß einer speziellen Ausgestaltung des erfindungsgemäßen Verfahrens können Wirtstiere, die für eine Passage des Parasiten verwendet wurden, auch zur Gewinnung von Antiserum herangezogen werden. In diesem Falle wird die Verabreichung von Eisenchelatbildner längere Zeit fortgesetzt, zweckmäßig wenigstens 10 Tage, vorzugsweise wenigstens 14 Tage nach der Infektion. Auf diese Weise behandelte Versuchstiere erweisen sich als gegenüber einer Neuinfektion durch den Parasiten immun und weisen bereits einen Antikörpertiter auf, der ihr Serum als Impfstoff verwendbar macht.

Erfindungsgemäß hergestellter Aktivimpfstoff weist eine so hohe antigene Wirksamkeit auf, daß er nicht nur zur Prophylaxe, sondern auch zur Therapie verwendet werden kann. Beispielsweise ist es möglich, einem malariakranken Patienten infizierte Erythrocyten zu entnehmen, diese unter Eisenmangelbedingungen in vitro zu züchten, schließlich die Erythrocyten zu lysieren und das erhaltene Lysat, welches die unter Hypoferrämiebedingungen gewachsenen Parasiten enthält, als Aktivimpfstoff dem gleichen Patienten wieder zu verabreichen.

Falls kein Aktivimpfstoff sondern ein Passivserum gewünscht wird, kann der mit dem erfindungsgemäß erhältlichen Antigen hergestellte Antikörper auch als monoklonaler Antikörper unter Anwendung der dem Fachmann
hierfür bekannten Methoden gezüchtet und als solcher
appliziert werden.

Die Erfindung wurde vorstehend unter Bezugnahme auf den
Malariaparasiten beschrieben. Sie eignet sich jedoch in
gleicher Weise auch zur Herstellung von Impfstoffen
gegen andere parasitäre Krankheitserreger aus der
Gruppe der Protozoen. Ein Beispiel für derartige andere
Parasiten sind Trypanosomen.

Die folgenden Beispiele erläutern die Erfindung weiter.


B e i s p i e l  1

I.    Herstellung antigenen Zellmaterials von P. vinckei

NMRJ-Mäuse, 25 g, werden zur Entleerung ihres Fe-
Speichers 3 Tage mit einem biologischen Komplexbildner (10 mg Desferrioxamin subcutan täglich)
vorbehandelt und mit P. vinckei infiziert und mit
10 mg Desferrioxamin subcutan täglich weiterbehandelt. Am fünften Tage nach der Infektion werden
die Tiere getötet (Parasitämie ca. 10 bis 30 %)
und das malariainfizierte Blut zur Infektion von
drei Tage mit Desferrioxamin vorbehandelten Versuchstieren der zweiten Passage verwendet.

Ab der zweiten Tierpassage werden die Versuchstiere mit einer höheren Dosis von Desferrioxamin (10
mg morgens; 5 mg mittags) versorgt und in der
Nacht unter Eisenmangeldiät gehalten. Im ganzen
werden 10 Tierpassagen durchlaufen.

Der Serumeisengehalt der Versuchstiere in Passage
eins ist auf ca. 50 % der Normalwerte (ca. 60 mg%
entsprechend ungefähr 20 bis 25 % Fe-gesättigtem
Transferin und 75 bis 80 % Fe-freiem Transferin)
und in den folgenden Passagen auf ca. 30 mg Serumeisen entsprechend 15 bis 10 % Fe-Transferin und
85 bis 90 % Fe-freiem Transferin (freie Eisenbindungskapazität) erniedrigt unter den angegebenen
Bedingungen.

II. Parasitenisolierung und Membrangewinnung

Nach der zehnten Tierpassage werden die Versuchstiere getötet, das infizierte Blut entnommen, mit
Heparin und Trasylol versetzt, Serum und Zellen
getrennt und die parasitierten Erythrocyten von
den nicht infizierten Zellen mittels Percollgradienten (vgl. Abstracts 10. Internationaler Kongress über tropische Medizin und Malaria, Manila,
9. bis 15. Nov. 1980 Nr. 467, Seiten 289/290) getrennt.

Aus den so gewonnenen infizierten Erythrocyten
werden die Parasiten nach der in Z. Parasitenkunde
_58_, 151 bis 159 (1979) beschriebenen Methode mittels Lectinen freigesetzt, durch Zentrifugieren
von Erythrocytenbestandteilen abgetrennt und mittels SDS-Elektrophorese und Enzymmessungen auf
Verunreinigungen durch Wirtszellmaterial untersucht.

Die so isolierten Parasiten werden durch milde Scherkräfte (Ultraturax/Frenchpress) aufgebrochen und die Membranen über einen linearen Dichtegradienten (z. B. 0,25 M bis 2,2 M Sucrose, Puffer, Proteinaseinhibitor) in der Ultrazentrifuge (2 Stunden, 65 000 g) aufgetrennt. Alternativ kann die Auftrennung auch durch normales Zentrifugieren oder dektrophoretisch erfolgen.

Die Membranfraktion wird gewonnen, elektrophoretisch auf Reinheit überprüft, gewaschen und in einer Menge von 1 mg Membranprotein pro 1 ml Serum der Versuchstiere (Spendertiere des antigenen Materials) aufgenommen.

III. Immunisierung der Versuchstiere

Zur Immunisierung wird eine Gruppe von normalen, nicht vorbehandelten Versuchstieren mit dem nach II. erhaltenen Material (unter Fe-Mangelbedingungen gehaltene P. vinckei in Spendertierserum) immunisiert. Eine zweite Gruppe von Versuchstieren wird mit nicht unter Fe-Mangel gehaltenen Erregern als Kontrolle infiziert.

Die Immunisierung bzw. Infektion erfolgt durch eine intramuskulare (0,1 ml) und 2 bis 5 mal intrakutane Injektion (5 x 10 µl) im Abstand von jeweils einer Woche.

Folgende Immunisierungsergebnisse werden erhalten:
1. mit nach II erhaltenem Material

    a) mit Vollblut       +++

    b) mit infizierten Erythrocyten alleine (ohne Serumzusatz)     ++

    c) mit Serum infizierter Tiere alleine     -

d) mit Erythrocytenmembranen parasitierter
Erythrocyten in Serum (Verunreinigung mit
Parasitenmaterial ca. 20 bis 40 %)                    -

e) mit isolierten Parasiten in Wirtsserum
(Verunreinigung durch Erythrocytenbestandteile unter 10 %)                                   +++

f) mit isolierten Parasitenmembranen in Serum
des Spendertiers

2. Kontrolle
mit nicht unter Fe-Mangelbedingungen gehaltenen Parasiten                                       -


B e i s p i e l   2


Spraque-Dawley Ratten bis 100 g Körpergewicht werden
mit P. vinckei, dem Erreger der Nagetiermalaria infiziert und mit 2 bis 10 mg Desferrioxamin subcutan pro
Tag behandelt. Die unbehandelten infizierten Kontrolltiere sterben etwa 5 bis 6 Tage nach der Infektion und
weisen dann eine Parasitämie von 70 bis 90 % und einen
Hämatokrit von 6 bis 10 % auf. Bei den mit Desferrioxamin behandelten Tieren liegt die Parasitämie zu diesem
Zeitpunkt zwischen 10 und 30 %, bei nur geringfügig erniedrigtem Hämatokrit. Wird die Behandlung zum Zeitpunkt
des Todes der Kontrollgruppe bei den behandelten Versuchstieren abgebrochen, so versterben die Tiere nach
weiteren 3 bis 4 Tagen mit einer hohen Parasitämie.

Wird die Behandlung mit Desferrioxamin weitergeführt,
so zeigen sich nach etwa dem 6. Tag die Symphtome einer
mittelschweren Parasitämie. Ab dem 9. bis 10. Tag nach
der Infektion gesunden die Tiere unter der fortgesetzten Desferrioxaminbehandlung schnell und nach 14 Tagen
ist die eigentliche Infektion abgeklungen. Bei Einstellen der Desferrioxaminbehandlung nach 14 Tagen tritt
keine Neuinfektion auf.

Werden die Versuchstiere, bei denen die Infektion nach
14 Tagen Desferrioxaminbehandlung abgeheilt ist, erneut
infiziert, so tritt keine Infektion auf. Die Tiere sind
immun.

Eine Gruppe von Versuchstieren wird mit einer Resochindosis behandelt, bei der sich über 14 Tage lang eine
der Desferrioxamin behandelten Gruppe vergleichbare Parasitämie entwickelt. Nach Überschreiten der 14tägigen
Infektionsdauer heilt die Infektion hierbei nicht von
alleine ab. Die Tiere erhalten eine kurative Dosis
Resochin, bis keine Infektion mehr feststellbar ist.
Dann wird die Resochingabe abgesetzt und die geheilten
Tiere werden erneut mit dem gleichen Erreger infiziert.
Dabei entwickelt sich erneut eine Infektion wie bei
nicht vorbehandelten Kontrolltieren. Dies zeigt, daß
auch eine ausgeheilte Infektion mit nicht erfindungsgemäß unter Hypoferrämiebedingungen gewachsenen Parasiten
keine Immunisierung bewirkt.

**Patentansprüche**

1. Verfahren zur Herstellung eines Impfstoffes gegen Parasiten, insbesondere Malariaparasiten, durch Infektion eines Organismus mit den Parasiten als Antigen und Gewinnung von Antiserum aus dem infizierten Organismus, **d a d u r c h g e k e n n z e i c h n e t ,** daß man mit dem Parasiten infizierte Erythrocyten in vivo oder in vitro unter Hypoferrämiebedingungen wachsen läßt, die Parasiten aus den Erythrocyten isoliert und die isolierten Parasiten oder Zellwandfragmente derselben als Antigen verwendet.

2. Verfahren nach Anspruch 1, **d a d u r c h g e k e n n z e i c h n e t ,** daß man Hypoferrämiebedingungen durch Zusatz eines Eisenchelatbildners bewirkt.

3. Verfahren nach Anspruch 2, **d a d u r c h g e k e n n z e i c h n e t ,** daß man einen biologischen Eisenchelatbildner verwendet.

4. Verfahren nach Anspruch 3, **d a d u r c h g e k e n n z e i c h n e t ,** daß man als biologischen Eisenchelatbildner Desferrioxamin verwendet.

5. Verfahren nach Anspruch 3, **d a d u r c h g e k e n n z e i c h n e t ,** daß man als biologischen Eisenchelatbildner Endotoxin, Ferritin oder/und Lactoferrin verwendet.

6. Verfahren nach einem der vorhergehenden Ansprüche, d a d u r c h g e k e n n z e i c h n e t , daß man den Erythrocyten oder deren Wirtsorganismus weniger Eisen als zur normalen Entwicklung benötigt, zuführt.

7. Verfahren nach einem der vorhergehenden Ansprüche, d a d u r c h g e k e n n z e i c h n e t , daß man hypoferrämische Parasiten bzw. deren Zellwandfragmente in Kombination mit hypoferrämischem Serum als Antigen verwendet.

8. Verfahren nach Anspruch 7, d a d u r c h g e k e n n z e i c h n e t , daß man dem Serum einen Proteinaseinhibitor zusetzt.

9. Verfahren nach einem der vorhergehenden Ansprüche, d a d u r c h g e k e n n z e i c h n e t , daß man das hypoferrämische Wachstum des Parasiten über eine Mehrzahl von Passagen fortführt.

10. Verfahren nach Anspruch 9, d a d u r c h g e k e n n z e i c h n e t , daß man nach jeweils 2 bis 8 Passagen den Eisenchelatbildnerzusatz erhöht.

11. Verfahren nach einem der vorhergehenden Ansprüche, d a d u r c h g e k e n n z e i c h n e t , daß man bei Züchtung des Parasiten in vivo das Wirtstier vor der Infektion ein- oder mehrere Tage unter Hypoferrämiebedingungen hält.

12. Verfahren nach einem der vorhergehenden Ansprüche, d a d u r c h g e k e n n z e i c h n e t , daß man die hypoferrämischen Bedingungen mindestens 14 Tage nach der Infektion aufrecht erhält.